# DEMANDE DE BREVET EUROPEEN

(11) **EP 2 243 460 A1**
(43) Date de publication de la demande: **27.10.2010**
(21) Numéro de dépôt: 10004276.1
(22) Date de dépôt: 22.04.2010
(51) Int. Cl.: A61J 7/04

(54) **Procédé et système d'enregistrement et de contrôle en temps réel du suivi d'un traitement médicamenteux**

(30) Priorité: 23.04.2009 FR 0901991
(71) Demandeur: ABR Pharma Société par actions simplifiée, 75002 Paris (FR)
(72) Inventeur: Bousquet, Bernard, 75005 Paris (FR)
(74) Mandataire: Benech, Frédéric

(57) **Abrégé**

La présente invention concerne un procédé et un système de contrôle en temps réel ou quasi réel du suivi d'un traitement médicamenteux pris sous forme de pilules par un patient. On détecte la prise de pilules par ledit patient à l'aide d'une plaquette équipée d'un circuit électronique de détection et d'enregistrement du moment d'extraction des pilules appartenant à un blister, on émet en temps réel à partir dudit circuit des signaux radio-électriques correspondant aux dites prises, on capte au fur et à mesure lesdits signaux que l'on transfert en temps réel ou quasi réel, à un serveur distant pour constituer un fichier de données d'observance dudit médicament associé audit patient, et on traite lesdites données d'observance pour contrôler, améliorer et/ou modifier le suivi du traitement par le patient.

## Description

La présente invention concerne un procédé d'enregistrement et de contrôle en temps réel ou quasi-réel du suivi d'un traitement médicamenteux pris sous forme de pilules délivrées sous blister par un patient, du type associé à l'utilisation d'une plaquette équipée d'un circuit électronique de détection et d'enregistrement du moment (date et heure) d'extraction de pilules appartenant au blister.

Elle concerne également un système de contrôle de l'observance mettant en oeuvre un tel procédé.

Outre ses applications en recherche clinique, elle trouve une application particulièrement importante, bien que non exclusive, dans le domaine de la mesure, du suivi, de l'amélioration et de la surveillance de l'usage des médicaments de prescription (ou observance) du patient ambulatoire.

Plus précisément, l'observance est le degré de respect par des patients ambulatoires d'une prescription médicamenteuse faite par un médecin, de ville ou hospitalier, puis délivrée par un pharmacien, d'officine le plus souvent, ou hospitalier dans le cas de médicaments qui réglementairement ne peuvent pas être distribués en pharmacie de ville.

Par blisters (dénomination anglo-saxonne désormais usuelle en français) on entend des plaquettes thermoformées munies d'alvéoles contenant des pilules c'est-à-dire au sens large des unités thérapeutiques solides, comprimés, gélules... , lesdites alvéoles ayant leur orifice fermé par une feuille ou un film (en général en aluminium). Ce film de protection se rompt plus ou moins facilement quand l'utilisateur extrait la pilule, le plus souvent en la poussant, pour forcer son passage au travers du film de protection.

On connaît déjà des procédés et dispositifs permettant d'enregistrer des données historiques sur les prises médicamenteuses par un patient.

Ils sont utilisables dans le cadre des études expérimentales, en recherche clinique, où le médicament est remis ou donné au patient par le médecin investigateur, sans passer par une prescription préalable décidée et proposée par un médecin, puis suivie par une délivrance en pharmacie.

De tels dispositifs et procédés sont non adaptés à un usage de routine pour une pratique quotidienne ou dans le cadre d'études de pharmacoépidémiologie dites « non interventionnelles » ou « faiblement interventionnelles ».

Ils ne permettent par ailleurs pas de transmettre des informations propres à influer en temps réel ou quasi-réel sur le comportement du patient qui serait, par exemple, défaillant pour prendre son médicament selon les recommandations du médecin.

Par temps quasi-réel on entend dans les minutes, voire dans les quelques heures qui suivent la détection d'une non conformité de prise ou encore d'une non prise en compte d'informations qui seraient communiquées au patient pour influer sur son comportement si celui-ci met du temps à réagir.

On connaît également (US 4,660,991) un procédé de mesures avec un dispositif de stockage et de signalisation périodique de prises de médicaments comprenant un émetteur de signaux sonores en forme de boîtier rigide muni d'une feuille amovible comprenant des obturateurs électriques permettant d'enregistrer la sortie d'une pilule par craquage d'un blister.

Un tel dispositif présente des difficultés de connectique (faux contacts), nécessite un boîtier assez encombrant, et est à l'origine de nombreuses erreurs.

Il ne permet en aucun cas à un patient d'apporter un réel correctif à un mauvais respect de sa part de la posologie.

De façon générale, les solutions connues utilisent des dispositifs à circuits électriques peu fiables, par exemple réalisés par l'impression d'encres aqueuses sur du papier lourd à fabriquer et posant des problèmes de connectique avec la partie électronique.

De telles solutions qui a priori ont nécessairement pour finalité, un usage expérimental exclusif, c'est à dire réservé à la recherche clinique en raison de la technologie utilisée, ne s'affranchissent souvent pas d'une opération de déconditionnement-reconditionnement des produits.

Celles-ci conduisent à modifier l'aspect d'un médicament pour le mettre sous le blister adéquat ce qui impose la réalisation préalable, quand elle est possible, d'une étude de stabilité du médicament toujours coûteuse.

Rappelons que le déconditionnement du médicament, nécessite soit l'intervention d'un tiers pour effectuer ces opérations, soit l'intervention du patient.

Dans les deux cas la solution n'est pas satisfaisante.

L'intervention d'un tiers impose des autorisations administratives préalables souvent impossibles à obtenir hors d'un cadre purement d'expérimentation ou de pilote.

Quant à l'intervention du patient, il s'agit d'une contrainte encore plus inacceptable pour les agences sanitaires, seul l'usage dans un contexte de recherche clinique étant autorisé sur le plan réglementaire.

En résumé, il n'y a pas aujourd'hui de procédé ou de système réellement fiable pour monitorer en temps réel chez un patient ambulatoire, le respect d'une posologie instituée par un médecin lors de la prescription d'un médicament (comprimé ou gélule) distribué sous blister

Or on attend d'une molécule ou d'une association de molécules, correspondant à un principe actif et prescrite en situation de vie réelle, un effet thérapeutique similaire à l'effet pharmacologique observé en situation expérimentale clinique où le respect de la prescription est généralement bien meilleure.

Dans la pratique, l'effet attendu ne correspond cependant jamais à l'effet observé en recherche clinique.

En dehors de raisons du type : erreurs de diagnostiques, autres erreurs dans le choix du au patient lui même (notamment un défaut du respect des contre-indications), erreurs dans la détermination de la posologie... la principale raison d'une sous ou non-efficacité d'un traitement médicamenteux chronique est le non-respect par le patient des consignes de prise données par le médecin : c'est donc un problème d'observance difficile à repérer même par un médecin sensibilisé et rompu aux problèmes d'observance médicamenteuse.

Ce problème est fondamental et n'a jamais été réglé correctement à ce jour.

Or le non respect des consignes de prise données par le médecin est la source potentielle d'une diminution de la sécurité entrainant une perte d'efficacité du médicament voire même d'une dangerosité de ce dernier.

Il s'agit donc d'un problème de santé publique largement identifié depuis longtemps par les autorités sanitaires qui, malgré leurs recommandations réitérées pour l'usage en vie réelle des produits, n'a pas été résolu.

En d'autres termes l'expérience montre que l'impact de santé publique d'un médicament (c'est-à-dire le rapport bénéfice/risque d'un médicament sur une communauté) est bien moindre que ne le laisserait prévoir l'efficacité mesurée dans des conditions expérimentales, l'une des raisons étant que le médicament n'est pas pris correctement.

Un des buts de la présente invention est donc d'améliorer l'impact de santé publique d'un médicament, notamment grâce à une gestion continue de son utilisation par le patient, de façon à rapprocher l'efficacité observée en vie réelle de celle obtenue dans les études expérimentales où, du fait de la formation spécifique des médecins investigateurs et du choix de patients identifiés comme bons observants, le respect des prescriptions est toujours meilleur.

Par exemple, s'agissant de l'amlodipine présentée à titre de paradigme, antihypertenseur normalisant la pression artérielle des sujets hypertendus, les études cliniques mettent en évidence une efficacité chez plus de deux patients sur trois.

Dans la vie réelle, un patient sur trois seulement est correctement pris en charge par l'amlodipine sous sa forme galénique actuelle. Un des objectifs de l'invention proposée est donc de permettre une augmentation de ce taux en le rapprochant du taux observés dans les études expérimentales.

Un grand nombre de patients hypertendus sont considérés par leur médecin comme des hypertendus résistants, c'est-à-dire des patients qui ne répondent pas pharmacologiquement aux traitements médicamenteux.

La pharmacorésistance n'est souvent en fait qu'un déficit d'observance que l'on peut tenter de corriger s'il est identifié en tant que tel.

Comme le souligne toutes les recommandations internationales consacrées à l'Hyper Tension artérielle (HTA), la première chose à faire, face à un patient hypertendu considéré comme pharmacologiquement résistant, est donc plutôt que de changer le traitement ou la posologie appliqués, de contrôler son observance médicamenteuse, ce qui était jusqu'à présent très difficile voire illusoire en pratique réelle.

La présente invention vise donc à pallier les inconvénients de l'art antérieur en ce qu'elle propose un procédé et un système de contrôle fiable, avec « feedback », en temps réel ou quasi-réel du suivi d'un traitement médicamenteux pris sous forme de pilules délivrées en blister, par un patient ambulatoire, répondant mieux que ceux antérieurement connus aux exigences de la pratique, dans le domaine de la recherche clinique mais aussi et surtout en dehors de ce domaine, et ce en utilisant les médicaments sous blisters du commerce.

La solution proposée, qui met en oeuvre un dispositif spécifique et un suivi à distance du médicament, interactif, avec « feedback » possible de la prise du médicament par le patient, ne présente pas de risque, est peu contraignante pour les patients, d'utilisation facile et intuitive, et ce de façon fiable.

Elle n'engendre pratiquement aucun risque de faux positifs ou de faux négatifs, et ce quelque soit les dimensions et/ou les formes des blisters et/ou des pilules tout en permettant de corriger le traitement en cours de prise.

Elle est indépendante de l'habilité du manipulateur, qui doit pouvoir être un patient à la vision non totalement corrigée et/ou présentant un trouble cognitif non diagnostiqué, ou encore des troubles articulaires, moteur, ou neurologiques, un horodatage de chaque prise médicamenteuse par étiquetage par le patient lui-même de ses blisters médicamenteux.

Dans ce but, l'invention propose notamment un procédé d'enregistrement et de contrôle en temps réel ou quasi réel du suivi d'un traitement médicamenteux pris sous forme de pilules par un patient,
**caractérisé en ce que**
on détecte la prise de pilules par ledit patient à l'aide d'une plaquette équipée d'un circuit électronique de détection et d'enregistrement du moment d'extraction des pilules appartenant à un blister, ladite plaquette étant formée d'un support souple sur laquelle est imprimé la partie conducteur dudit circuit pour former des interrupteurs propres à être ouverts lorsque les pilules sont extraites manuellement par un utilisateur, après collage du blister sur ladite plaquette,
on émet en temps réel à partir dudit circuit des signaux radio-électriques correspondant aux dites prises,
on capte au fur et à mesure lesdits signaux que l'on transfert en temps réel ou quasi-réel, (par exemple via une centrale de transfert situé dans l'environnement immédiat ou accompagnant le patient) à un serveur distant pour constituer un fichier de données d'observance dudit médicament associé audit patient,
et on traite lesdites données d'observance pour contrôler, améliorer et/ou modifier le suivi du traitement par le patient.

Avantageusement la plaquette étant autoadhésive, et le dispositif comportant des moyens de guidage du collage du blister sur la plaquette, celle-ci est collée par le patient sur le blister. Ainsi donc à partir de simples plaquettes/étiquettes autoadhésives positionnées par un patient éventuellement aidé par un système de guidage les résultats obtenus sur l'observance sont particulièrement exceptionnels.

Dans des modes de réalisation avantageux on a de plus recours à l'une et/ou à l'autre des dispositions suivantes :
- on capte les signaux par le biais d'un dispositif téléphonique connecté directement ou indirectement au réseau Internet ;
- on traite automatiquement les données d'observance obtenues à partir d'un algorithme de contrôle déterminé et on retransmet au patient en temps réel ou quasi réel et au vu de l'observance ou non par le patient de son traitement, les actions à entreprendre par ce dernier.

Eventuellement, un traitement des données d'observance peut également se faire en local sur un PDA, un téléphone multi fonctions ou un micro-ordinateur.

Un logiciel de traitement, d'analyse et de « feed back » pour mettre de l'intelligence est alors prévu en local.

Les actions à entreprendre peuvent être transmises directement ou par un intermédiaire (médecin, infirmière, pharmacien ou autres professionnels de la santé, la famille, des aidants... ).
- on retransmet les actions à entreprendre au patient concerné de façon automatique ;
- cette retransmission est effectuée par des message de texte ou un appel téléphonique ;
- on demande de façon répétitive au patient concerné un signal correspondant à l'action correctrice jusqu'à celui-ci soit délivré ;
- on trie et on analyse les différents fichiers d'observance pour identifier et repérer de façon automatique ou semi-automatique les patients concernés par un problème potentiel ou advenu lié aux dites observances et/ou aux types de patients.

La présente invention propose également un système de suivi mettant en oeuvre le procédé décrit ci-dessus.

Elle propose aussi un système de contrôle en temps réel ou quasi réel du suivi d'un traitement médicamenteux pris sous forme de pilules par un patient, **caractérisé en ce qu**'il comporte au moins un dispositif de mesure de l'observance, ledit dispositif comprenant une plaquette équipée d'un circuit électronique de détection et d'enregistrement du moment d'extraction des pilules appartenant à un blister et des moyens de fixation par collage du blister à la plaquette, la plaquette étant formée d'un support souple sur laquelle est imprimée la partie conducteur dudit circuit pour former des interrupteurs propres à être ouverts lorsque les pilules sont extraites manuellement par un utilisateur, ledit système comportant des moyens d'émission en temps réel à partir dudit circuit des signaux radio-électriques correspondant aux dites extractions,
des moyens de captation au fur et à mesure desdits signaux et de transfert en temps réel ou quasi-réel à un serveur distant pour constituer un fichier de données d'observance dudit médicament associé audit patient,
et des moyens de traitement desdites données d'observance associés audit serveur distant pour contrôler, améliorer et/ou modifier le suivi du traitement par le patient.

Avantageusement on a de plus recours à l'une et/ou à l'autre des dispositions suivantes :
- les moyens de traitement associés au serveur distant des dites données comprennent des moyens de stockage de références correspondant à au moins une plaquette dans un premier fichier constitué lors de l'émission d'un premier signal reçu correspondant à ladite plaquette répertoriée dans un deuxième fichier, des moyens de stockage desdits signaux correspondant à ladite plaquette ou à un jeu de plusieurs plaquettes en vue du suivi de l'observance dans un troisième fichier, formant le fichier de données d'observance dudit médicament pour un patient répertorié dans un quatrième fichier,
   des moyens de calcul automatique du suivi correct ou non de son traitement par le patient au vu des données des premier, deuxième, troisième et quatrième fichiers,
   des moyens de formation d'un cinquième fichier d'instruction au patient à partir des résultats de calcul et
   des moyens de transmission automatique ou semi automatique de ce cinquième fichier à distance au patient ;
- le système comporte de plus des moyens agencés pour utiliser Internet pour interroger et dialoguer avec le serveur à distance pour consulter s'il y a lieu un ou plusieurs des premier, deuxième, troisième, quatrième et/ou cinquième fichiers ;
- les moyens d'émission en temps réel à partir dudit circuit des signaux radio-électriques correspondant aux dites prises comportent un dispositif téléphonique connecté directement ou indirectement au réseau Internet ;
- le système comporte de plus des moyens de tri et d'analyse des différents fichiers des données d'observance pour identifier et repérer de façon automatique ou semi-automatique les patients concernés par un problème potentiel ou advenu lié aux dites observances et/ou aux types de patients.
   Il est aussi possible d'en sortir des statistiques et/ou modifier des traitements ultérieurs ;
- le circuit électronique de détection d'un dispositif comporte des portions conductrices identiques réparties régulièrement en parallèle le long d'un chemin conducteur, ledit chemin conducteur étant à chaque fois agencé pour présenter une plus grande résistance que la portion en parallèle, le support comprenant des portions frangibles oblongues sur lesquelles sont imprimées ces portions conductrices réparties régulièrement pour être sensiblement au droit d'une pilule correspondante de sorte que la portion frangible de la plaquette et la portion conductrice correspondante sont rompues simultanément lorsqu'une pilule est extraite, le circuit comportant de plus des moyens de mesure de l'impédance globale de la partie conducteur pour en déduire le moment de l'extraction d'une pilule.

Avantageusement la plaquette est formée d'un support souple sur lequel est imprimé par dépôt ou collage une partie métallique conductrice dudit circuit pour former les interrupteurs.

L'impression doit être comprise au sens de circuit « imprimé ».

Le chemin conducteur est quant à lui et par exemple formé d'un fil ou film longiligne, d'épaisseur faible (quelques microns ou dizaines de microns) et de largeur par exemple comprise entre 0,1 et 0,5 mm, par exemple 0,2mm.

Par support souple on entend un support d'une grande flexibilité comme peut l'être une feuille de papier mais suffisamment résistant dans le sens de son plan pour ne pas autoriser de cassure par un utilisateur peu précautionneux tout en permettant de le déformer sur lui même de façon courbe ou ondulée.

Avantageusement on utilisera pour support une feuille mince (< 0,1 mm, par exemple 0,05 mm) totalement souple et indéchirable, en Polyéthylène Naphtalate (PEN), ou encore formé d'un polyimide ou de polyester ou tout autre support souple en feuille de matière plastique ou autre, formant un complexe compatible avec un contact avec un médicament.

Dans un mode avantageux le circuit comporte des portions conductrices identiques, réparties régulièrement, et doublées au niveau de chaque interrupteur par une autre chemin conducteur offrant une résistance calibrée.

La plaquette est de plus avantageusement transparente, suffisamment pour permettre la lecture des mentions figurant sur les films protecteurs des blisters.

Les patients étant souvent assujettis à suivre un traitement avec plusieurs médicaments et pouvant présenter un déficit visuel, il est important de ne pas créer un risque de confusion entre deux médicaments.

Mais surtout, dans le mode de réalisation plus particulièrement décrit ici, elle comprend des portions frangibles intégrant le bord, c'est à dire à cheval sur le bord ou situées directement en vis à vis d'une partie des portions conductrices en forme de languette formant interrupteur. Lesdites portions frangibles sont ainsi réparties régulièrement sur les zones de sortie d'une pilule correspondante de sorte que la portion frangible de la plaquette sensiblement en périphérie d'une portion conductrice est rompue lorsqu'une pilule du blister fixée en vis-à-vis est extraite.

Autrement dit par portions frangibles au bord des portions conductrices, on entend des portions conductrices à cheval sur la périphérie c'est à dire en partie à l'intérieur de la zone frangible, par exemple en formant une boucle connectée de chacun de ses côtés, avec le reste de la partie conductrice située à l'extérieur de ladite zone.

Avantageusement, les moyens de mesure de l'impédance globale comportent d'une part des moyens repérant les moments d'incrémentation de l'impédance globale de la partie conducteur et d'autre part des moyens de calcul agencés pour en déduire le moment d'extraction d'une pilule.

Egalement avantageusement le circuit comporte des moyens agencés pour éliminer ou minimiser encore plus considérablement les risques de prise en compte de faux contacts, avant de conclure à l'éjection d'une pilule.

Ces moyens sont avantageusement des moyens de calculs agencés pour retester régulièrement l'impédance globale de façon à s'affranchir des faux positifs et/ou négatifs, c'est à dire donc et comme on l'a déjà dit, l'enregistrement d'une sortie de pilule ou de non sortie de pilule à tort.

A noter que le terme mesure d'impédance est utilisé dans l'invention de façon générale et peut en fait être ramené à une mesure de la résistance du circuit, avec une pile d'alimentation en courant continu.

Le principe consistant à mesurer la résistance de l'ensemble d'un circuit qui, petit à petit, voit son impédance augmenter, par palier correspondant à la valeur calibrée de chaque résistance mise en parallèle, au fur et à mesure que les médicaments sont extraits, et ce, quelque soit la pilule extraite au début ou à la fin de la chaîne, permet ainsi une mesure fiable et simple grâce à un logiciel intégré dans le circuit, logiciel dont la conception est à la portée de l'homme du métier, au vu du problème posé.

Du fait de l'utilisation d'un circuit imprimé métallique, la fiabilité de la mesure est renforcée. En effet il est ainsi possible d'utiliser des résistances électroniques dont la valeur est précise, calibrée, invariable d'une résistance à l'autre et stable dans le temps ce qui est essentiel pour bien identifier le nombre de pilules prises en cas d'expulsion de plusieurs pilules de façon quasi simultanée.

La disposition des portions conductrices au bord des zones frangibles, hors des surfaces d'arrachement du film fermant les alvéoles, généralement en aluminium et potentiellement conducteur, entraînent par ailleurs, en combinaison avec le reste des caractéristiques, une impossibilité ou quasi impossibilité de court-circuit.

Dans un mode de réalisation avantageux les portions frangibles comportent des trous, orifices ou évidements oblongs, c'est à dire ovales ou plus généralement non circulaires barrés d'une languette.

Dans un mode de réalisation avantageux, l'impossibilité de court-circuit est renforcée par la présence d'un vernis transparent ou le rajout sur la plaquette d'un matériau isolant, faisant partie des moyens complémentaires pour éliminer la prise en compte de faux contact.

Avantageusement les moyens de fixation par collage sont formés d'une couche de colle protégée par un film pelable avant fixation du blister sur la plaquette.

Comme précisé ci-avant et dans un mode de réalisation avantageux de l'invention plus particulièrement décrit ici, on a vu que pour éviter de manière encore plus fiable que toute tentative infructueuse d'extraction d'une pilule ne s'accompagne de l'action d'un interrupteur, on prévoit un retestage régulier de l'impédance globale.

Pour se faire on mesure par exemple périodiquement l'impédance, ou la tension aux bornes du circuit, ce qui revient au même, puis on moyenne les valeurs obtenues sur n mesures pour obtenir une tension moyenne Tᵢ, que l'on compare avec la valeur de tension moyenne précédente Tᵢ-₁.

Si cette valeur Tᵢ-Tᵢ₋₁ est supérieure à une valeur seuil s de tension, on en déduit la sortie d'une pilule.

En d'autres termes, les moyens pour retester périodiquement et/ou régulièrement l'impédance globale comprennent des moyens de comparaison d'au moins une première tension moyenne Tᵢ sur n mesures, avec une tension moyenne précédente Tᵢ₋₁ sur n mesures.

Avantageusement, n est compris entre 5 et 10, par exemple 8, et/ou au moins deux couples de tensions moyennes consécutifs sont vérifiés pour valider une sortie de pilule.

Avec des interrupteurs irréversibles, fiables et hautement frangibles, et de surcroît ne nécessitant pas un procédé de contrôle qualité trop important, générant un surcroît de coût, il est ainsi possible d'obtenir un résultat sans pratiquement aucun faux.

L'invention sera mieux comprise à la lecture de la description qui suit de modes de réalisation donnés ci-après à titre d'exemples non limitatifs.

La description se réfère aux dessins qui l'accompagnent dans lesquels :
La figure 1 est montre schématiquement un système pour mise en oeuvre du procédé selon un mode de réalisation de l'invention.
La figure 2 montre un autre mode de réalisation d'un système selon l'invention.
La figure 3 donne une vue en plan d'un mode de réalisation d'une plaquette appartenant à un dispositif utilisé dans un système selon l'invention.
Les figures 3A et 3B illustrent deux modes de réalisation de portions frangibles et de portions conductrices utilisables avec l'invention.
Les figures 4 et 5 sont des vues de dessus de variantes de circuits utilisables avec l'invention.
Les figures 4A et 5A sont des vues agrandies schématiques des portions de circuit respectivement des figures 4 et 5 au droit d'une portion frangible correspondante.
La figure 6 est une vue schématique représentant les moyens de traitement du serveur distant pour contrôler, améliorer et/ou modifier le suivi du traitement.
La figure 7 est un schéma bloc montrant un mode de réalisation du test anti-faux positifs, mis en oeuvre dans un mode de réalisation selon l'invention.

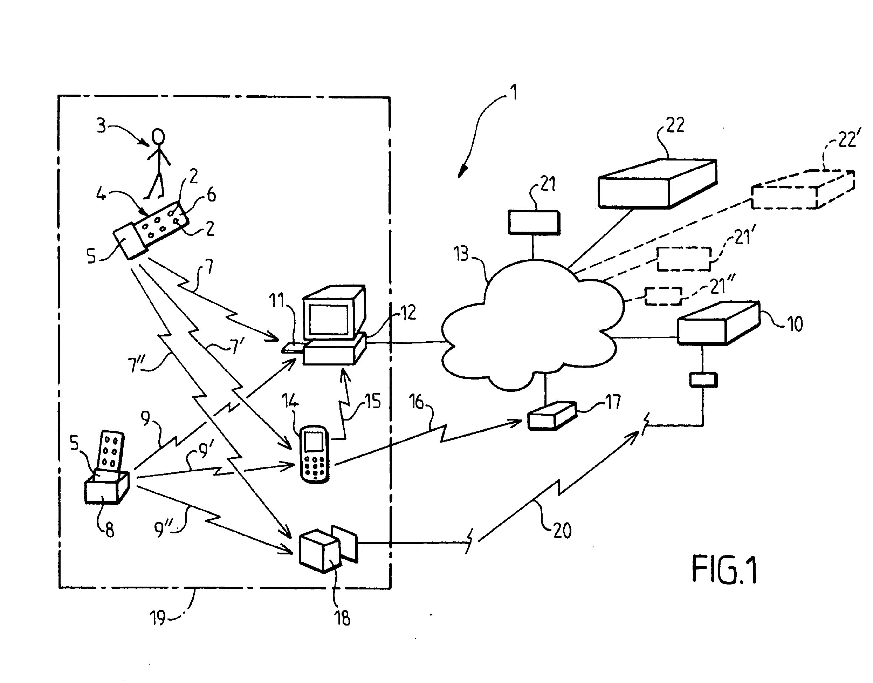

La figure 1 montre un système 1 pour mise en oeuvre du procédé d'enregistrement et de contrôle en temps réel du suivi d'un traitement médicamenteux pris sous forme de pilules 2 par un patient 3.

La prise de pilules 2 est détectée à l'aide d'une plaquette 4 équipée d'un circuit électronique 5 (ou étiquette) de détection et d'enregistrement du moment d'éjection des pilules appartenant à un blister 6 collé sur la plaquette.

Lors de l'éjection d'une pilule, on émet en temps réel à partir du circuit 5, des signaux radio-électriques (flèches 7, 7', 7") correspondant auxdits prises, ou on stocke cette information qu'on émet en temps différé (quasi-réel) en passant par un support 8 dans lequel on insert le circuit 5 (flèches 9, 9', 9').

Le but est de transmettre à un serveur à distance 10 les informations nécessaires pour le suivi de l'observance du patient 3 c'est-à-dire pour traitement en vu du contrôle, de l'amélioration et/ou la modification dudit suivi par le patient.

Ces informations sont essentiellement une identification du circuit 5 ainsi que les données d'horodatage de la ou des prises médicamenteuses qui viennent d'être effectuées.

Dans tous les cas, il y a une transmission à partir de chaque étiquette.

Celle-ci se fait à chaque fois qu'une prise de pilule est enregistrée, ou encore, selon un scénario paramétrable permettant de l'adapter à chaque traitement, ou encore de façon différée pour assurer la bonne exécution du transfert des données et/ou économiser l'énergie.

La transmission se fait sous forme d'un fichier (numéro d'identification unique de l'étiquette électronique + données d'horodatage), par exemple sur la bande de fréquence à 2,4 GHz, vers une « centrale locale » de transfert du fichier vers le réseau Internet.

La centrale locale est toujours équipée d'un récepteur/émetteur captant les fichiers émis par les étiquettes et comprend des moyens permettant une transmission comme décrit ci-après.

La transmission se fait par exemple, mais de façon non limitative :
- soit par une clé USB 11 connectée à un ordinateur local 12 relié à Internet 13 (flèches 7 et 9),
- soit par le biais d'une carte mini SD (pour mini Secure Digital Card) en mode dit mini SDIO, qui permet d'utiliser des périphériques d'entrée/sortie avec une carte mini SD (flèches 7', 9').

Cette carte insérée dans un PDA ou un téléphone intelligent (« smart phone » en terminologie anglo-saxonne) 14, permet le transfert (flèches 15 ou 16) via ce dernier en temps réel vers le serveur distant 10, via Internet 13 en local (flèche 15) ou via une borne externe 17.
- soit par un module GSM/modem 18 associé qui doit rester dans l'environnement (trait mixte 19) du patient 3, et qui permet une transmission par exemple directe (flèche 20) vers le serveur distant 10.

L'ensemble de ces modules et/ou un de ces modules permettant le transfert vers le serveur distant, forme(nt) la centrale locale.

Dans le mode de réalisation plus particulièrement décrit le serveur 10 peut dialoguer avec d'autres serveurs et/ou ordinateurs 21, 21', 21", ... connectés au réseau Internet 13 ou à un réseau intranet, pour permettre au médecin traitant, aux services de l'hôpital, etc, de connaître le fichier d'observance de leurs patients en instantané ou quasi-instantané, et d'y réagir éventuellement.

Les données transmises sont cryptées et agencées pour respecter le secret médical.

Comme on l'a vu l'observance s'analyse au regard de la concordance entre une prescription et les prises de médicaments réelles par un patient selon des paramètres variés :
- pourcentage de la dose prise par rapport à la dose prescrite pendant la durée de la prescription,
- prises omises,
- prises en dehors des horaires recommandés par la prescription,
- jours avec prises surnuméraires,
   et en cas de non-conformité de prise :
- conformité ou non de la procédure de rattrapage si des recommandations ont été faites par le médecin

On donne ci-après de façon non limitative plusieurs exemples d'algorithmes de suivi, d'autres algorithmes étant également donnés plus loin dans la description pour illustrer encore mieux les applications de l'invention.

Exemple 1 : si une patiente oublie un comprimé d'Arimidex® un jour, elle ne doit en prendre qu'un le lendemain.

Exemple 2 : si un patient oublie de prendre son antihypertenseur le matin, il peut encore le prendre le soir et continuer le lendemain de façon normale.

Exemple 3 : il existe un nouvel anticancéreux, si un patient a oublié de prendre son traitement le matin, il peut le prendre jusqu'à 15 heures mais pas après puis prendre à nouveau normalement son traitement le lendemain.

Avantageusement un ou plusieurs serveurs 22, 22', ... de tri et de rassemblement des données (qui peuvent être confondus avec le serveur 10 ou les serveurs 21, 21',...), sont prévus pour collecter l'ensemble des informations, ici encore de façon cryptée et en respectant la confidentialité et le secret médical. La collecte de ces données va permettre d'établir statistiques et pronostics.

Les serveurs 22 sont par exemple des ordinateurs de sociétés pharmaceutiques uniquement intéressées par leurs propres médicaments.

D'autres modes de transmission sont bien entendu possibles comme par exemple le simple téléphone filaire du patient (non représenté). Une puce récepteur est alors intégrée au téléphone/modem branché sur le réseau téléphonique filaire du patient.

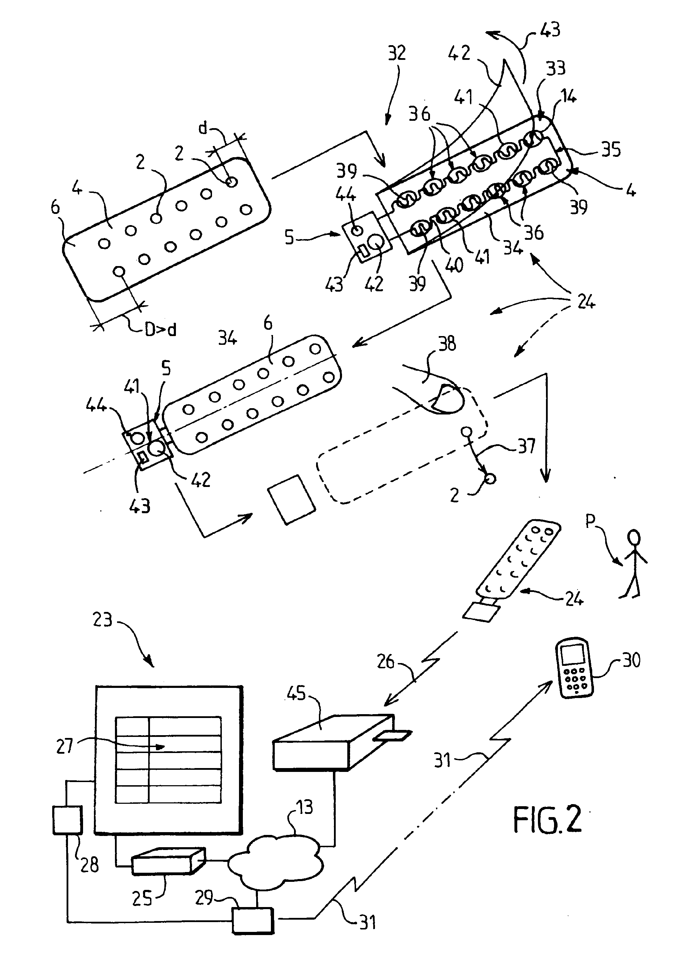

La figure 2 montre plus précisément un des modes de réalisation d'un système 23 de contrôle en temps réel ou quasi-réel selon l'invention.

A partir d'au moins un dispositif 24 de mesure de l'observance, on récupère en temps réel ou quasi-réel sur un serveur distant 25 les signaux 26 émis par le dispositif 24 pour constituer un fichier 27 de données d'observance du médicament concerné associé au patient P ayant utilisé le dispositif.

En plus des moyens de constitution d'un tel fichier 27, le système comprend des moyens 28 de traitement desdites données d'observance pour contrôler, améliorer et/ou modifier le suivi du traitement du patient et des moyens 29 de transmission des résultats partiels ou totaux du traitement en retour au patient par exemple par Internet et/ou sur son téléphone portable 30 (flèche 31).

Les moyens de traitement et de transmission sont constitués de façon connue en elle-même par les micro processeurs et/ou modems adéquats, ou par tous autres moyens à la portée de l'homme du métier.

L'analyse et le traitement sont chaque fois faits selon un algorithme dédié prenant en compte les caractéristiques pharmacocinétiques du traitement, les recommandations médicales en vigueur dans le pays des patients, les notices de médicament, l'AMM, les caractéristiques du patient, la prescription du médecin, les recommandations du médecin en charge du patient...

On a donné ci-après à nouveau trois exemples (non limitatifs) d'algorithmes dédiés.

Exemple 1 : Il s'agit d'un médicament à prendre tous les jours, dont la prescription peut ne pas être suivie sans trop de gravité, tel un hypocholestérolémiant.

Dans ce cas, un « feedback » ne devient nécessaire et efficace qu'en cas d'oubli de plus de deux jours ou d'oublis répétés avec insuffisamment de prises compensatoires les jours suivant des jours sans prise. Avantageusement on peut modéliser la pharmacocinétique du médicament chez le patient pour limiter les interventions au plus strictement utile.

Exemple 2 : Il s'agit d'un médicament de l'ostéoporose, connu sous la marque « Fosamax® », à prendre une fois par semaine mais 30 mn avant le petit déjeuner.

Si les premières prises montrent que la patiente prend son traitement régulièrement à 9 heures, le « feedback » sera donné en cas d'oubli constaté à 10 heures (c'est à dire à l'heure probable de son petit déjeuner).

Le jour où la patiente doit prendre son traitement, le message suivant est par exemple émis : « Ne prenez pas votre comprimé aujourd'hui. Prenez le demain au moins 30 mn avant votre petit déjeuner, puis la semaine prochaine le jour où vous prenez habituellement votre traitement ».

Exemple 3 : Dans le cas d'anticoagulant comme celui connu sous la marque « Pradaxa®», la posologie est de deux comprimés le matin (médicament prescrit à la sortie de l'hôpital pendant les 30 jours qui suivent la pose d'une prothèse de hanche chez une patiente).

Ici, du fait de la gravité et de la fréquence du risque d'embolie pulmonaire en l'absence de traitement, si dans les premiers jours de traitement, on constate que le Pradaxa® est régulièrement pris à 9 heures, le « feedback » sera systématique en cas de déviation (un seul comprimé à la place de deux, oubli de la double prise constaté à 10 heures, prise surnuméraire).

Le retour d'information est quant à lui et de façon non limitative effectuée selon les habitudes et l'environnement technologique du patient : utilisation de son téléphone portable ou de son filaire, email, fax...

Quand on utilise son téléphone portable ou filaire, cela peut faire l'objet de : SMS, d'un appel avec message préenregistré (serveur vocal), d'un appel émanant d'un centre d'appel qui peut, ou ne peut pas, intégrer les compétences de professionnels de santé ... etc.

Dans des modes de réalisation, il est prévu la mise en place d'un site collaboratif qui peut être sur un serveur 21, où le médecin peut entrer de façon sécurisée, des données concernant le patient, sa prescription... le médecin pouvant encore plus personnaliser l'algorithme d'analyse et de traitement.

Le site collaboratif permet ici de mettre l'ensemble des données en consultation facile et permanente pour le médecin, le patient et autres professionnels.

Dans le mode de réalisation plus particulièrement décrit en référence à la figure 2, on utilise un circuit électronique de détection et d'enregistrement qui comprend une puce émetteur/récepteur, ce qui permet de faire communiquer entre elles toutes les étiquettes que l'on donne à un patient.

L'intérêt est ici que ces puces se transmettent l'ensemble des données d'horodatage concernant le patient.

Ainsi si un patient voyage, s'il existe des problèmes de communication... toutes les données d'horodatage peuvent facilement être récupérées puisque consolidées sur chaque étiquette.

L'ensemble des étiquettes d'un même patient va alors constituer un réseau dont l'architecture sera le fruit d'un compromis entre l'objectif d'économie d'énergie, la fiabilité de la conservation et transmission des données, l'immédiateté de la transmission...

De plus, dans un mode de réalisation particulier de l'invention, plusieurs traitements médicamenteux d'un même patient peuvent être gérés simultanément en tenant compte d'éventuelle interaction. Il existe des traitements où l'efficacité du produit A est conditionné par la prise simultanée du produit B. Dans ce cas extrême, la non-prise de B pourrait aboutir à l'instruction suivante : « le patient devrait arrêter de prendre A car il ne prend pas B ».

A noter que pour les patients qui utiliseraient la carte miniSDIO ou la clef USB intégrant la puce utilisée essentiellement comme récepteur, une solution utilisant un logiciel en local peut être prévue autorisant un traitement et l'analyse des données localement.

Dans la suite de la description on utilisera les mêmes numéros de références pour désigner les mêmes éléments ou des éléments similaires.

La figure 2 montre également les autres éléments qui sont mis en oeuvre avec un système selon l'invention, dans son mode de réalisation plus particulièrement décrit ici.

Le dispositif 24 de mesure de l'observance comprend la plaquette 4 équipée du circuit 5 électronique de détection et d'enregistrement du moment d'éjection des pilules 2 appartenant au blister 6.

Le blister 6 est formé de façon connue d'une feuille souple comprenant par exemple deux rangées parallèles de six pilules 2.

Le dispositif comprend des moyens 33 (colle laminée sur la face supérieure de la plaquette) de fixation du blister à la plaquette. La plaquette est formée d'un support souple 34 en PEN sur lequel est imprimée la partie conducteur 35 du circuit électronique pour former des interrupteurs 36 propres à être ouverts lorsque les pilules sont éjectées manuellement (voir flèche 37) par un utilisateur dont le pouce 38 est représenté sur la figure 2.

Plus . précisément, le circuit électronique 5 comporte des portions conductrices 39 identiques réparties régulièrement le long du chemin conducteur 40.

Le support est donc souple, flexible, transparent, sensiblement rectangulaire, et comprend des portions frangibles 41, oblongues, en bordure desquelles sont imprimées les portions conductrices 39, réparties régulièrement pour être sensiblement au droit d'une pilule 2 correspondante lorsque le blister 6 est fixé sur la plaquette, de sorte que la portion frangible de la plaquette et la portion conductrice correspondantes sont rompues simultanément lorsqu'une pilule est éjectée.

La partie conductrice est constituée d'un aplat cuivré étamé adhérent à son support souple de 0,2 à 0,5 mm de largeur.

Comme indiqué ci-avant, le support transparent est un support flexible type PEN (Polyéthylène Naphtalate), par exemple, un PEN de 50 microns d'épaisseur sur lequel on fait laminer (coller) un feuillard de cuivre de 15 à 35 microns.

Le PEN est très résistant et transparent. S'il y a des inscriptions à ne pas masquer sur la surface plane d'un blister, on s'arrange par ailleurs pour faire le tracé en fonction de celle-ci.

Le circuit 5 comporte des moyens 41' de mesures de l'impédance globale de la partie conducteur pour en déduire le moment de l'extraction des comprimés ou pilules.

Ces moyens 41' comportent une pile d'alimentation électrique 42, une puce 43 électronique de mesure programmée en conséquence et un ensemble 44 de constituants électroniques : mémoire, quartz, résistance et antenne à radiofréquence (RFId) notamment .

Cet ensemble 44 comprend également une puce émetteur/récepteur (bande de fréquence de 2,4 GHz par exemple) avec un mode de gestion à très basse consommation d'énergie.

Les moyens 41' sont constitués d'éléments connus, raccordés et programmés de façon à la portée de l'homme du métier.

Les moyens 33 de fixation sont formés d'une couche de colle protégée par un film plastique 42 pelable (flèche 43) avant fixation du blister 6 sur la plaquette.

Avantageusement des moyens de guidage (non représentés) sont prévus. Ils sont par exemple formés par un carton épais, évidé, de la dimension du dispositif, dans lequel ce dernier vient s'encastrer et par dessus la plaquette préalablement pelée duquel le blister peut également venir s'encastrer à son tour dans le reste de la hauteur de l'évidemment,.

Egalement avantageusement une couche d'isolant électrique (non représentée) et/ou de résine ou de vernis isolant, est ajoutée au préalable entre la couche de colle et la plaquette.

Ainsi, quand le patient prend une pilule 2, la date et l'heure exacte sont enregistrées par la puce 43 dans la mémoire 44.

Selon l'invention une centrale de transfert à radiofréquence 45, lisant et interprétant les ondes radio électromagnétique 26, est prévue, capte et transmet en temps réel ou quasi-réel les fichiers au serveur distant 25.

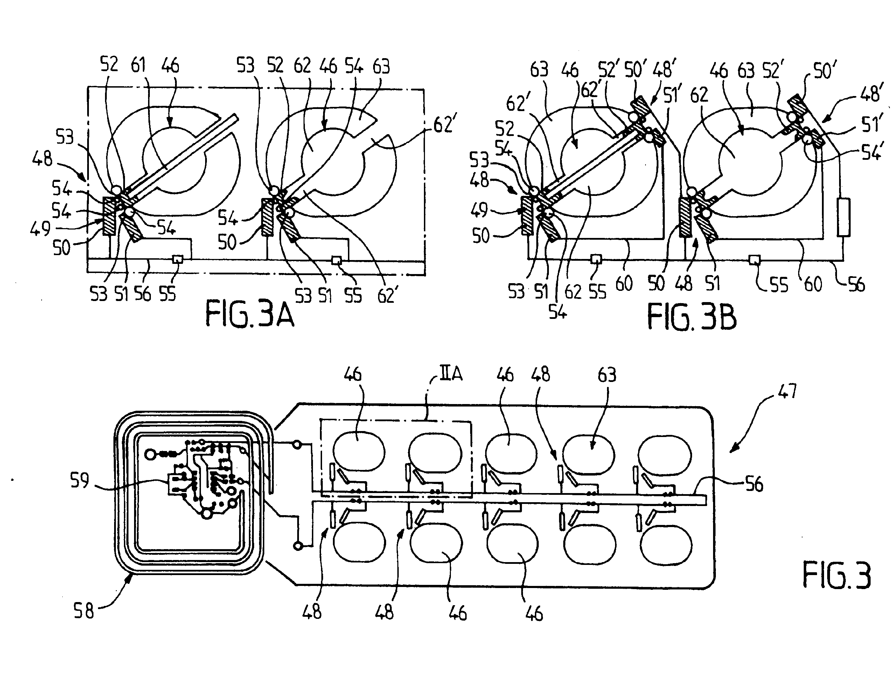

On a représenté sur la figure 3 un autre mode de réalisation d'une plaquette 47 (utilisable avec un système) selon l'invention.

Ici les portions conductrices 48 situées juste à la périphérie de chaque orifice d'extraction des pilules du blister, formées par des évidements oblongs ou ovales 46 dans la plaquette, et propres à être rompues lors de chaque extraction d'une unité thérapeutique par un utilisateur, sont formées (cf. figures 3A et 3B) par des pattes 49 à deux bras 50 et 51 conductrices, de plus grosse largeur que le fil conducteur de liaison, par exemple de cinq à dix fois plus large, reliées entre elles par un portion intermédiaire 52 (située dans la portion frangible) et détachables l'une de l'autre, par brisure de leur deux zones 53 de jonction avec la portion 52, fragilisées par des perforations 54 réalisées à leur niveau dans la plaquette.

Des résistances 55 ramenées sur le chemin conducteur 56, en parallèle avec les portions conductrices 48 bloquent le passage, et obligent le courant à passer par l'intermédiaire des pattes à deux jambes détachables et des zones de jonction 53.

Dans un mode de réalisation, ces résistances peuvent être ramenées à proximité des moyens de calculs à l'extrémité de la plaquette, ce qui entraîne alors la nécessité de multiplier les chemins conducteurs sur la plaquette.

Lorsqu'une rupture se fait, le courant est amené à passer par la partie plus résistante du circuit (résistance 55), ce qui entraîne une augmentation ou incrémentation de l'impédance, correspondant à la valeur d'une résistance, mesurée par le circuit électronique et la puce (non représentée) connectés à l'extrémité 58 de la plaquette via la portion 59 de circuit imprimé de façon connue en elle-même.

Les moyens de calcul du circuit sont protégés par un boîtier en matière plastique plat, sensiblement carré, par exemple de 40 à 50 mm de côté, muni de moyens d'encliquetage par petits ergots des deux faces creuses l'un sur l'autre, deux des ergots, étant par ailleurs insérés dans des trous d'extrémité de la plaquette ou feuille de support du reste du circuits, ce qui rend cette dernière indésolidarisable du boîtier même quand on tire dessus.

Plus précisément, et par exemple on a d'un côté la partie souple autoadhésive de l'étiquette et de l'autre la partie électronique.

Les composants sont quant-à eux soudés sur un support dur non souple.

Celui-ci est par exemple une plaque rigide d'époxy/fibre de verre de 8/10 mm d'épaisseur. La connexion entre la partie souple en PEN et la partie supportant l'électronique a été résolue en soudant un connecteur ZIP sur la partie dure.

On enfiche la partie souple dans ce connecteur. L'avantage de l'existence de ce connecteur est de permettre de réutiliser la partie électronique.

Dans un mode de réalisation avantageux (figure 3B), chaque portion conductrice 50 est formée par deux sous-portions 48 et 48' formant deux interrupteurs disposés en série (liaison 60) de part et d'autre de la portion frangible 57 correspondante, de forme oblongue, de dimensions plus importantes que celles des pilules correspondantes à extraire et/ou de leurs alvéoles de rétention dans le blister correspondant.

La sous portion 48' est similaire à la sous portion 48, et comprend deux bras 50' et 51', reliées par une portion intermédiaire 52' craquable, car fragilisée comme la sous portion 48.

Ainsi que la rupture du blister se fasse d'un côté ou de l'autre, le risque de défaut positif est encore minimisé.

L'interrupteur placé en périphérie de l'orifice de sortie de la pilule qui lui correspond, formé par les parties conductrices 48, en parallèle du chemin conducteur principal 56, comme représentée sur les figures 2A et 2B permet, en combinaison avec les moyens complémentaires pour éliminer la prise en compte de faux contacts, d'éliminer ou de minimiser ces risques de faux contacts pouvant éventuellement advenir dans le cas fréquent où le blister comporte une feuille métallique conductrice de fermeture.

En effet, associé au fait que les parties conductrices sont du côtés de la plaquette opposé au côté en contact direct avec le blister, le positionnement excentré fait qu'on limite le risque d'avoir une portion du circuit (formé par exemple par un fragment de cuivre étamé), qui rentre en contact avec le blister.

L'avantage du positionnement excentré est aussi de faciliter en général la réalisation du cheminement de la partie conductrice qui est opaque.

Avec le présent concept de plaquette (étiquette) il est possible de disposer de plusieurs options : l'option excentrée, l'option comme on va le voir en U, l'option où on fait une simple amorce de déchirure par une découpe à vif sans finir par un trou....

Pour un même blister, il va être ainsi possible d'utiliser pour les pilules les plus éloignée l'option U et l'option traversante pour les pilules les plus proches du connecteur.

Dans un mode de réalisation, une zone 61 formée par une encre, ou un fil cuivré, avantageusement placé de l'autre côté de la plaquette, au verso du circuit, permet de renforcer la partie centrale 62, placée dans l'évidement 63 de la plaquette, sur le passage de la pilule, ce qui évite tout risque de rupture ailleurs qu'au niveau des interrupteurs 48.

La partie centrale 62 est adaptée à chaque série différente de blisters. Elle est par exemple circulaire, reliée au reste de la plaquette par deux bras opposés 62', dont les extrémités comportent les parties 52 et 52' propres à être rompues, et occupe entre la moitié et le quart de la surface de l'évidement 63.

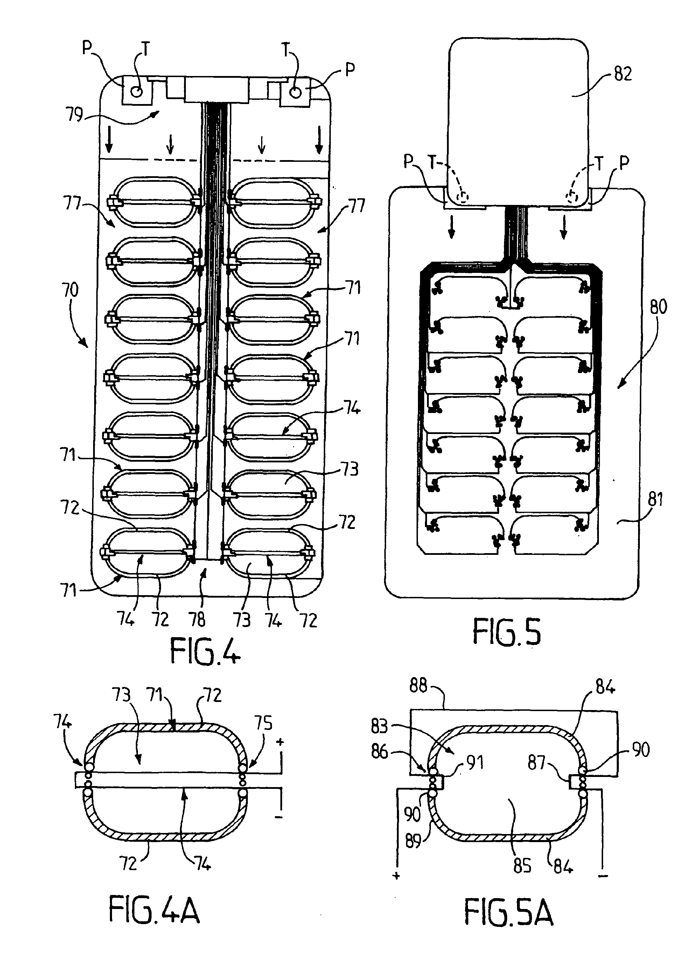

On a représenté sur les figures 4, 4A et 5, 5A deux autres modes de réalisation d'élément d'un dispositif selon l'invention, de façon non limitative.

La figure 4 montre le chemin conducteur 70 d'un circuit électrique selon un autre mode de réalisation de l'invention.

Ici les évidements oblongs 71 comprennent deux zones de découpe 72 de part et d'autre, laissant une grande languette ovale 73 au centre, correspondant à l'orifice de sortie de la pilule.

La portion conductrice en parallèle 74 est montrée agrandie sur la figure 4A. Elle est formée par exemple par une trace de cuivre de 0,2 mm de largueur.

Dans ce mode de réalisation elle présente une forme en U dont les deux branches forment une boucle qui traverse la languette 73 en présentant à chaque extrémité et à cheval sur le bord de l'évidement 71, des zones de rupture 75, obtenues par des petits trous ronds 76 par exemple de 1,2 mm de diamètre avec un espacement par exemple de 0,3 mm.

Les zones de découpe 72 prennent en compte et corrigent le décalage possible dans le positionnement de l'étiquette par rapport au blister.

Une telle disposition en U présente l'avantage d'économiser la place sur les côtés 77 de la plaquette en feuille 78, présentant sa partie connectique 79 avec le boîtier (non représenté) qui contient l'électronique embarquée.

Il y a de plus quatre interrupteurs au lieu de deux, ce qui multiplie les risques de cassures grâce aux trous différents.

La figure 5 montre schématiquement un autre mode de réalisation du chemin conducteur 80 d'un dispositif selon l'invention.

Seul le chemin conducteur est ici représenté sur la feuille 81 en matière plastique type PEN propre à être fixée au boîtier 82 par le biais d'une pinoche interne

Plus précisément les moyens de fixation entre boitier et feuille de plastique sont mécaniques et assurent une grande solidité de jonction entre eux. Ils sont par exemple formés par deux pinoches en excroissance situées sur la partie interne de la moitié du couvercle dudit boitier, qui traversent la feuille 78 ou 81 de part en part par le biais de trous T en vis à vis, percés dans des renforts carré ou petites plaques P situées au bord de ladite feuille, (voir figure 4) pour s'encastrer dans des orifices correspondant de l'autre moitié du couvercle, les deux moitiés étant de plus et par exemple fixées l'une avec l'autre par collage.

La figure 5B montre plus précisément un évidement 83, muni comme pour le mode de réalisation précédent de parties évidées 84 entourant une languette transparente ovale 85.

Toute la zone de la languette 85 restant transparente, on peut lire plus facilement au travers, ce qui est avantageux pour certains blisters pour lesquels des informations importantes sont imprimées au centre de l'alvéole contenant la pilule.

Ici le chemin présente deux fois deux interrupteurs 86 et 87, de part et d'autre, le chemin électrique 88 passant à l'extérieur de l'évidement.

Les points de fragilisation du chemin 88, à cheval sur le bord 89 de l'évidement sont par exemple formés par des petits trous 90, comme décrit précédemment par exemple deux plus gros trous, séparés par deux plus petits, le chemin 91 passant entre les trous, les deux plus petits étant situés entre les deux fils qui forment donc la boucle 91.

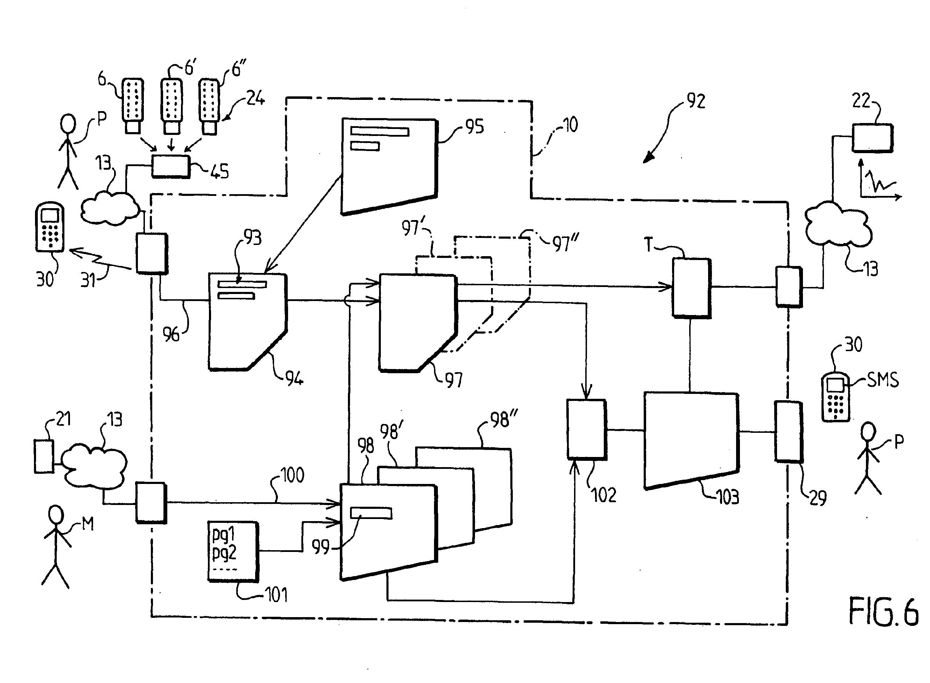

On a représenté sur la figure 6 un mode de réalisation des moyens 92 de traitement du serveur distant 10 (trait mixte) pour contrôler, améliorer et/ou modifier le suivi du traitement d'un patient P selon l'invention.

Le patient P dispose par exemple de plusieurs dispositifs 24 auxquels sont collés les blisters 6, 6', 6" correspondant au traitement qu'un médecin traitant M lui a prescrit.

Il est muni d'un téléphone portable 30, les dispositifs 24 pouvant transmettre les données directement à un modem 45 local, relié par Internet 13 au serveur 10.

Le médecin M dispose quant à lui et par exemple d'un ordinateur 21 connectable par le réseau Internet 13 au serveur 10.

Plus précisément les moyens 92 de traitement du serveur distant 10 du système comprennent des moyens de stockage des références 93 correspondant à une plaquette dédiée à un blister 6 déterminé dans un premier fichier 94 (voir exemple Table I) constitué lors de l'émission du premier signal reçu, correspondant à ladite plaquette à partir d'un deuxième fichier de référence 95, et des données d'horodatage 96 obtenues du patient.

Le fichier 94, qui est un fichier tampon d'arrivée des réponses dans le temps permet de constituer le fichier 97 (97', 97" , ... ) formant le fichier des données d'observance du médicament pour un patient répertorié dans un quatrième fichier 98, 98', 98", associé à une posologie 99 déterminée par le médecin M (lien 100) et/ou correspondant à des programmes pré-enregistrés en 101.

Des calculs (moyens 102) du suivi correct du traitement par le patient sont alors effectués à partir des premier, deuxième, troisième et quatrième fichiers et permettant la formation d'un cinquième fichier 103 d'instruction au patient à partir des résultats de calcul.

Ceux-ci sont alors transmis automatiquement ou semi-automatiquement au patient P par exemple par SMS.

Avantageusement des moyens T de tri et de calcul permettent de transmettre par Internet des éléments divers, de statistique notamment, à un autre serveur 22 comme décrit ci-avant.

**TABLE I**

| | | | | | |
|---|---|---|---|---|---|
| Plaquette | N°1 | Médicament X | Nb de pilules | Date de péremption | Maladie traitée |
| Plaquette | N°2 | - | - | - | - |

On a donné ci-après des exemples de traitement pour contrôler, améliorer et/ou modifier le suivi du traitement par le patient, et permettant d'élaborer de façon à la portée de l'homme du métier les algorithmes et programmes informatiques qui vont générer les instructions aux médecins et/ou au patient pour ledit suivi.

Pour de nombreux médicaments, qui, comme Arimidex®, sont prescrits à la posologie de une prise par jour, la proportion de jours avec un oubli de prise peut être importante et classiquement supérieure à une fois par mois pour plus de la moitié des patients.

En cas d'oubli sur un jour unique, on constate souvent la prise de deux comprimés le lendemain alors que la recommandation du médecin est de un comprimé par jour même pour le jour qui suit un oubli.

Pire, en cas d'oubli pendant deux jours consécutifs, la prise de trois comprimés en une prise unique le jour suivant est loin d'être une exception et est proscrite par les recommandations. Avec l'horodatage automatisé des prises au moment de chaque prise et une restitution des données au médecin lors de la consultation suivante, le médecin peut apporter des conseils bien ciblés et -orientés. Mais avec les mêmes capacités d'horodatage associées à la transmission en temps réel à un autre serveur et à un centre de traitement comme avec l'invention, il est possible de déterminer des modalités d'action et de monitorage du patient en temps réel.

Selon les cas (selon le profil pharmacocinétique du médicament, selon les préférences du médecin, selon le niveau de santé du patient, selon son niveau de compréhension et d'acceptation du traitement, selon l'environnement technologique et de communication du patient... ), les actions possibles sont par exemple:
- pour les médicaments type antihypertenseur à la posologie de une prise le matin et où en cas de non prise le matin, il est encore possible de prendre son médicament le soir, l'action possible est l'envoi d'un message au patient en début d'après midi pour qu'il prenne le médicament oublié ;
- pour un médicament anticancéreux à la posologie de une prise toutes les 12 heures et pour lequel l'intervalle entre deux prises ne doit pas être inférieur à 6 heures au risque d'induire une baisse de la sécurité d'emploi, l'action possible est, tous les jours où il est constaté un oubli à 15 heures, l'envoi au patient d'un message clair pour qu'il reprenne son traitement normalement le soir mais que surtout, il ne prenne pas deux comprimés ;
- pour un médicament antiagrégat plaquettaire, lorsqu'il est constaté que le patient effectue des prises erratiques, l'action possible est : l'envoi d'un message quotidien au patient afin qu'il prenne bien son traitement. Le message est envoyé quelque instant avant l'heure habituelle de la prise (et n'est pas envoyé, bien sûr, si la prise a déjà eu lieu). Si le patient prend trop souvent des prises surnuméraires, une réponse graduée peut être envisagée : un message d'alerte simple au patient puis si le patient met en danger sa vie, le message d'alerte peut être directement envoyé au médecin.

Comme indiqué précédemment, le canal d'envoi des messages est adapté aux patients : SMS, email, fax, messages téléphoniques préenregistrés, appel téléphonique par un téléopérateur, contact par un professionnel de santé... Tout est possible en fonction de la technologie auquel le patient accède.

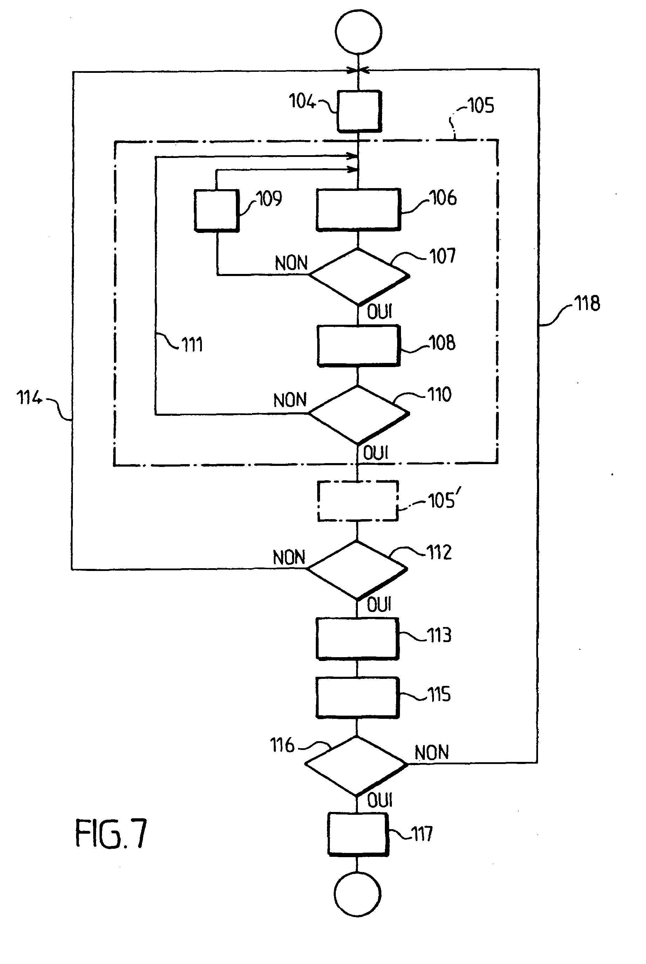

On va maintenant décrire en référence à la figure 7 les moyens de calculs et plus particulièrement de tests selon un mode de réalisation de l'invention permettant d'éviter les faux positifs et/ou faux négatifs de façon extrêmement efficace.

On initialise tout d'abord dans un premier temps en 104 le processus de suivi.

Puis, est réalisé un cycle 105 (trait mixte) de mesures de tension, de la façon suivante. On mesure en 106 de façon périodique, par exemple toutes les secondes, la tension T du circuit du dispositif.

Lorsque le nombre de mesures est inférieur à n (test 107), on stocke la mesure en 109, et on recommence le test.

Si le nombre de mesure devient égal à n, on effectue en 108 la moyenne des tensions T indice i sur les n mesures à partir des mesures stockées en 109, valeur moyenne Tᵢ que l'on stocke alors dans une mémoire tampon du circuit électronique inclus dans le boîtier en plastique.

On effectue alors le test 110 dans lequel on compare les moyennes de T indice i et T indice i-1.

Si cette comparaison montre que cette valeur est inférieure à une valeur seuil déterminée au préalable, on effectue un nouveau cycle de tests identique au cycle 105 en 105', sinon (ligne 111), on revient à la mesure 106 périodique de la tension.

Dans le cas où la deuxième mesure moyennée de tension est toujours différente de la tension indice i moins 1 (test 112) au delà de la valeur seuil, on considère qu'il y a effectivement prise d'une pilule et on stocke (bloc 113) les éléments la concernant dans la mémoire des moyens de calcul. Sinon on réitère le processus en réinitialisant les séries de mesures en 104 (ligne 114).

Lorsque cette mesure de prise est stockée, elle est ensuite éventuellement soumise à différents calculs (blocs 115) qui vont pouvoir être par exemple copiés par le biais d'une lecture de la puce RFID sur un dispositif externe non représenté.

A la fin de l'utilisation de la plaquette (test 116) on transmet la plaquette en 117 au médecin pour analyse.

Sinon on réinitialise (ligne 118) le fonctionnement.

On va maintenant décrire plus précisément un exemple de réalisation du fonctionnement de l'élimination de faux positifs et/ou négatifs.

Pour se faire on a par exemple mis en mémoire trente seuils correspondant aux trente prises à effectuer sur une plaquette.

Trente prises est en général un nombre maximum de pilules pour les blisters du commerce.

Plus généralement les blisters comportent quatorze ou quinze pilules.

Pour se faire on mesure la tension aux bornes du circuit électronique, tension qui augmente au fur et à mesure des prises, car chaque fois qu'il y a une prise un interrupteur s'ouvre et le courant ne passe plus directement mais à travers une résistance supplémentaire.

En d'autres termes, le circuit augmente sa résistance (également qualifiée d'impédance) au fur et à mesure des prises.

La problématique que se propose de résoudre cet algorithme plus particulier est en fait d'éviter les faux positifs.

En effet, il a pu être observé qu'il y avait de temps en temps une mesure faussée dont la raison exacte était difficile à déterminer.

Celle-ci peut venir de court-circuits, le circuit étant peu protégé, par exemple juste par une couche de colle, et peut donc venir au contact de la partie aluminium du blister quand les interrupteurs sont rompus.

Par ailleurs, dans le cas d'une utilisation de puce RFID, le système peut détecter de fausses mises en communication et tirer également sur la consommation de la pile qui est incluse dans le dispositif électronique.

Cette pile étant une pile de faible puissance, elle peut alors éventuellement transmettre une tension insuffisante dans le circuit et simuler une prise ou une absence de prise alors que cette dernière a ou n'a pas eu lieu.

Quoiqu'il en soit cette identification de problèmes rencontrés dans le cadre d'une utilisation de blisters du commerce, par des utilisateurs normaux avec ce type de dispositif, est nouvelle.

Dans ce mode de réalisation l'invention le gère par des moyens tels que précisés ci-avant pris en combinaison, et notamment, dans le mode de réalisation plus particulièrement décrit, par des moyens de calcul algorithmiques mettant en oeuvre des tests successifs, le contrôle étant réalisé au moins une deuxième fois, éventuellement une troisième fois, etc.

On ne conclura à une prise que lorsque deux mesures successives de moyennes de T, c'est à dire en l'espèce dans l'exemple mentionné deux fois huit mesures moyennées, permettent de conclure consécutivement à la dite prise.

Avec le dispositif selon ce mode de réalisation de l'invention, on a ainsi pu constater un seul faux positif sur plus de six cent prises, la fiabilité pouvant encore être améliorée en augmentant le nombre de cycles 105 de contrôle.

Un autre avantage inattendu provient du fait que, si une connexion ou plusieurs se referment alors qu'il n'y a pas eu de nouvelles prises, par exemple du fait de la manipulation de la plaquette, cela ne crée pas d'erreurs puisque les prises sont stockées en mémoire.

On va maintenant décrire le fonctionnement du dispositif et du procédé selon le mode de réalisation de l'invention plus particulièrement décrit ici en référence à la figure 2.

L'utilisateur (patient) P à qui un médicament correspondant au blister 6 a été prescrit, se voit fournir, soit par son médecin, soit par le pharmacien, soit directement par le fabricant du blister, un dispositif 24 selon l'invention.

Celui-ci, fabriqué en grande série, a un coût faible, compte tenu notamment de la simplicité de la puce 41 dite RFID (initiales anglo-saxonnes de Radio-Frequency Identification) qui comporte les moyens de calcul, de stockage et de transmission des informations d'enregistrement.

L'utilisateur détache la pellicule 42 sur le dispositif qu'il colle ensuite sur le blister 6, sans se préoccuper du côté du blister plus long au moins long, compte tenu des surfaces frangibles oblongues.

Eventuellement des moyens de guidage comme décrits ci-avant, l'aide dans la mise en place du blister.

Le blister est alors collé, pilules vers le dessus, la face en feuille d'aluminium étant du côté du dispositif.

Grâce au fait que ce dernier est transparent, les indications figurant au dos du blister sont lisibles au travers de la plaquette 4.

L'utilisateur, au fur et à mesure de son traitement extrait ensuite les pilules 2 en pressant manuellement sur le blister.

En appuyant sur le blister, la pilule rigide craque le film d'aluminium et en même temps l'une et/ou l'autre des parties de jonction.

L'impédance du circuit est alors augmentée ce qui active le compteur et les enregistrements des prises médicamenteuses.

A chaque prise, le blister/dispositif émet les signaux correspondant à une telle prise vers le modem 45 d'interface par exemple, puis vers le serveur distant 25.

Celui-ci effectuant alors les traitements nécessaires pour constituer la fiche d'observance 27 du patient, qui va alors pouvoir être consultée par son médecin qui, de façon connue, interroge via son ordinateur, de façon à obtenir les données concernant les prises.

Le serveur 25 transmet par ailleurs via les différents moyens évoqués ci-avant le « feed-back » nécessaire au patient P.

Sachant qu'il a été monitoré de façon infalsifiable dans ses prises de médicaments, l'utilisateur voit sa conduite en temps réel ou quasi-réel corrigée pour qu'il puisse respecter la prescription, ce qui permet une plus grande efficacité du médicament, chez l'utilisateur déambulatoire.

Comme il va de soi et comme il résulte également de ce qui précède, la présente invention n'est pas limitée aux modes de réalisation plus particulièrement décrits. Elle en embrasse au contraire toutes les variantes et notamment celles où le boîtier qui contient les moyens de calcul est amovible et réutilisable, où les pilules sont des comprimés ou des gélules, ou plus généralement des unités thérapeutiques (ampoules... )ou éléments (seringues... ) conditionnés en blister.

L'étape d'analyse et de feed back peut également être plus sophistiquée.

Ainsi non seulement il peut y avoir un échange d'informations avec le médecin pour paramétrer le traitement des données, mais d'autres données comportementales du patient (historique de prise) et d'autres variables peuvent être couplées. Par exemple des paramètres mesurés par des dispositifs complémentaires comme la valeur de glycémie, la tension, le poids ... peuvent être inclus dans l'analyse et le traitement des données comportementales.

## Revendications

1. Procédé de contrôle en temps réel ou quasi réel du suivi d'un traitement médicamenteux pris sous forme de pilules par un patient, **caractérisé en ce que**
on détecte la prise de pilules par ledit patient à l'aide d'une plaquette équipée d'un circuit électronique de détection et d'enregistrement du moment d'extraction des pilules appartenant à un blister, ladite plaquette étant formée d'un support souple sur laquelle est imprimé la partie conducteur dudit circuit pour former des interrupteurs propres à être ouverts lorsque les pilules sont extraites manuellement par un utilisateur, après collage du blister sur ladite plaquette,
on émet en temps réel à partir dudit circuit des signaux radio-électriques correspondant aux dites prises,
on capte au fur et à mesure lesdits signaux que l'on transfert en temps réel ou quasi réel, à un serveur distant pour constituer un fichier de données d'observance dudit médicament associé audit patient,
et on traite lesdites données d'observance pour contrôler, améliorer et/ou modifier le suivi du traitement par le patient.

2. Procédé selon la revendication 1, **caractérisé en ce que**, on capte les signaux par le biais d'un dispositif téléphonique connecté directement ou indirectement au réseau Internet.

3. Procédé selon l'une quelconque des revendications précédentes, **caractérisé en ce que** on
traite automatiquement les données d'observance obtenues à partir d'un algorithme de contrôle déterminé et on retransmet en temps réel ou quasi réel et au vu de l'observance ou non par le patient de son traitement, les actions à entreprendre par ce dernier.

4. Procédé selon la revendication 3, **caractérisé en ce que** on retransmet les actions à entreprendre au patient concerné de façon automatique.

5. Procédé selon la revendication 4, **caractérisé en ce que** cette retransmission est effectuée par des message de texte ou un appel téléphonique.

6. Procédé selon l'une quelconque des revendications 3 à 5, **caractérisé en ce que** on demande de façon répétitive au patient concerné un signal correspondant à l'action correctrice jusqu'à celui-ci soit délivré.

7. Procédé selon l'une quelconque des revendications précédentes, **caractérisé en ce que** on trie et on analyse les différents fichiers d'observance pour identifier et repérer de façon automatique ou semi-automatique les patients concernés par un problème potentiel ou advenu lié aux dites observances et/ou aux types de patients.

8. Procédé selon l'une quelconque des revendications précédentes, **caractérisé en ce que** on teste régulièrement l'impédance pour détecter les faux positifs et/ou négatifs.

9. Procédé selon la revendication 8, **caractérisé en ce que** pour tester l'impédance on mesure périodiquement la tension Tᵢ puis on moyenne les valeurs obtenues sur n mesures, que l'on compare avec la valeur Tᵢ₋₁ obtenue par une moyenne précédente, et si cette valeur est supérieure d'une valeur s déterminée on en déduit l'extraction d'une pilule.

10. Procédé selon la revendication 9, **caractérisé en ce que** n est compris entre cinq et dix, et au moins deux couples de tensions moyennes successives sont vérifiées pour valider une sortie de pilule.

11. Système de contrôle en temps réel ou quasi réel du suivi d'un traitement médicamenteux pris sous forme de pilules par un patient, **caractérisé en ce qu'**il comporte des dispositifs de mesure de l'observance, chaque dispositif comprenant une plaquette équipée d'un circuit électronique de détection et d'enregistrement du moment d'extraction des pilules appartenant à un blister et des moyens de fixation du blister à la plaquette, la plaquette étant formée d'un support souple sur laquelle est imprimé la partie conducteur dudit circuit pour former des interrupteurs propres à être ouverts lorsque les pilules sont extraites manuellement par un utilisateur, ledit système comportant
des moyens d'émission en temps réel à partir dudit circuit des signaux radio-électriques correspondant aux dites prises,
des moyens de captation au fur et à mesure desdits signaux et de transfert en temps réel à un serveur distant pour constituer un fichier de données d'observance dudit médicament associé audit patient,
et des moyens de traitement desdites données d'observance associé audit serveur distant pour contrôler, améliorer et/ou modifier le suivi du traitement par le patient.

12. Système selon la revendication 11, **caractérisé en ce que** les moyens de traitement associés au
serveur distant des dites données comprennent des moyens de stockage de références correspondant à au moins une plaquette dans un premier fichier constitué lors de l'émission d'un premier signal reçu correspondant à ladite plaquette répertoriée dans un deuxième fichier, des moyens de stockage desdits signaux correspondant à ladite plaquette ou à un jeu de plusieurs plaquettes en vue du suivi de l'observance dans un troisième fichier, formant le fichier des données d'observance dudit médicament pour un patient répertorié dans un quatrième fichier,
des moyens de calcul automatique du suivi correct ou non de son traitement par le patient au vu des données des premier, deuxième, troisième et quatrième fichiers,
des moyens de formation d'un cinquième fichier d'instruction au patient à partir des résultats de calcul et
des moyens de transmission automatique ou semi automatique de ce cinquième fichier à distance au patient.

13. Système selon l'une quelconque des revendications 11 et 12, **caractérisé en ce que** il comporte de plus des moyens agencés pour utiliser Internet pour interroger et dialoguer avec le serveur à distance pour consulter s'il y a lieu un ou plusieurs des premier, deuxième, troisième ou quatrième et/ou cinquième fichiers.

14. Système selon l'une quelconque des revendications 11 à 13, **caractérisé en ce que** les moyens d'émission en temps réel à partir dudit circuit des signaux radio-électriques correspondant aux dites prises comportent un dispositif
téléphonique connecté directement ou indirectement au réseau Internet.

15. Système selon l'une quelconque des revendications 11 à 14, **caractérisé en ce que** il comporte de plus des moyens de trie et d'analyse des différents fichiers d'observance pour identifier et repérer de façon automatique ou semi-automatique les patients concernés par un problème potentiel ou advenu lié aux dites observances et/ou aux types de patients.

16. Système selon l'une quelconque des revendications 11 à 15, **caractérisé en ce que** le circuit comporte des portions conductrices identiques réparties régulièrement en parallèle le long d'un chemin conducteur, ledit chemin conducteur étant à chaque fois agencé pour présenter une plus grande résistance que la portion en parallèle, **en ce que** le support est souple, transparent, comprend des portions frangibles oblongues sur lesquelles sont imprimées ces portions conductrices réparties régulièrement pour être sensiblement au droit d'une pilule correspondante de sorte que la portion frangible de la plaquette et la portion conductrice correspondante sont rompues simultanément lorsqu'une pilule est extraite et **en ce que** le circuit comporte des moyens de mesure de l'impédance globale de la partie conducteur pour en déduire le moment de l'éjection d'une pilule.

17. Système selon l'une quelconque des revendications 11 à 16, **caractérisé en ce que** la plaquette comprend des portions frangibles intégrant le bord, c'est à dire à cheval sur le bord ou situées directement en vis à vis d'une partie des portions
conductrices en forme de languette formant interrupteur.

18. Système selon l'une quelconque des revendications 11 à 17, **caractérisé en ce que** chaque dispositif comporte des moyens complémentaires pour améliorer l'élimination de la prise en compte de faux contacts générant des faux positifs ou des faux négatifs.

19. Système selon la revendication 18, **caractérisé en ce que** les moyens complémentaires pour éliminer la prise en compte de faux contacts comprennent des moyens de calcul agencés pour retester régulièrement l'impédance globale de façon à écarter les risques de faux positifs.

20. Système selon la revendication 19, **caractérisé en ce que** les moyens pour retester périodiquement et/ou régulièrement l'impédance globale comprennent des moyens de comparaison d'au moins une première tension moyenne Tᵢ sur n mesures avec une tension moyenne précédente Tᵢ₋₁.
